# EUROPEAN PATENT APPLICATION

(11) **EP 2 100 622 A2**
(43) Date of publication of application: **16.09.2009**
(21) Application number: 09250658.3
(22) Date of filing: 09.03.2009
(51) Int. Cl.: A61L 2/07, A61L 2/24

(54) **An air detection circuit**

(30) Priority: 08.03.2008 GB 0804355
(71) Applicant: Eschmann Holdings Limited, Eschmann House, Peter Road Lancing West Sussex, BN15 8TJ (GB)
(72) Inventor: Spendley, David, Lancing West Sussex, BN15 8TJ (GB)
(74) Representative: Peel, James Peter

(57) **Abstract**

The present invention discloses an autoclave having an air detection circuit for use in detecting whether a pre-determined volume of air is present in a sample of steam wherein the autoclave comprises a chamber and a vacuum pump and wherein the air detection circuit is operatively connectable to the chamber and comprises:
an air detector having a separator for separating air from the sample of steam and a sensor for detecting whether the separated air has a predetermined volume;
wherein the autoclave is arranged such that the air detection circuit is operatively connectable to the vacuum pump such that the vacuum pump can be used to empty the air detection circuit. The present invention further relates to a method of detecting whether a pre-determined volume of air is present in a sample of steam.

## Description

The present invention provides for an improved method and apparatus to be used in detecting whether a pre-determined volume of air is present in a sample of steam. The invention also includes an autoclave associated with such apparatus.

In steam sterilization of articles, especially of medical devices, it is important to ensure that substantially all the trapped air in an autoclave is removed. Any air or non-condensable gases remained locally will prevent the required sterilization temperatures to be reached, thus compromising the sterilization. Manufacturers of autoclaves are constantly seeking for systems which can perform residual air test based on standard methods in order to ensure the conformity of their apparatus to the present legislation. Ideally, the test for the presence of air or non-condensable gases should be performed at the end of every sterilization cycle to offer the best assurance for the efficacy of the sterilization. In addition, the users of autoclaves prefer fully automatic systems which do not require the user intervention in the event of a failure in the sterilization cycle.

EP-A-1 867 344 to Eschmann describes a detection circuit comprising an air ingress detector connected to a condenser wherein the condenser is for receiving gases from the chamber of an autoclave. The detector is connected to a water waste tank through a restrictor. The restrictor is said to have a diameter sufficiently narrow so as to be able to maintain the pressure in the detector to be substantially the same as that in the chamber without being so narrow that the time taken for the condenser to drain is too long to be acceptable to a user of the autoclave. In practice, the restrictor feature sometimes was found not to work efficiently because the restrictor would not allow the detector to empty after a detection cycle. In view of this, it may be difficult to prime the detector by re-filling it with water.

A way of ameliorating these potential problems has been sought.

According to the invention there is provided An autoclave having an air detection circuit for use in detecting whether a pre-determined volume of air is present in a sample of steam wherein the autoclave comprises a chamber and a vacuum pump and wherein the air detection circuit is operatively connectable to the chamber and comprises:
an air detector having a separator for separating air from the sample of steam and a sensor for detecting whether the separated air has a predetermined volume;
wherein the autoclave is arranged such that the air detection circuit is operatively connectable to the vacuum pump such that the vacuum pump can be used to empty the air detection circuit.

According to the invention there is also provided a method of detecting the presence of a pre-determined volume of air in a sample of steam, comprising the steps of:
providing an air detector adaptable to detect whether a predetermined volume of air is present;
emptying the contents, if any, in the air detector;
passing a sample of steam through the air detector;
separating any air from the sample of steam in the air detector; and
detecting whether the separated air has the pre-determined volume.

One of the advantages of the autoclave according to the invention and of the method of the invention is that the air detector can be cleared of any residue from a prior use in detecting the presence of a pre-determined volume of air before being used again. This is important for ensuring that the air detector of the air detection circuit is emptied of air between uses to prevent erroneous reading that a successful sterilisation cycle was a failure due to presence of residual air in the air detector.

In some embodiments, the method of the invention further comprises the step of cooling a sample of steam before the passing step. In some embodiments, the autoclave according to the invention comprises a condenser for cooling the sample of steam. One advantage of cooling the sample of steam or including a condenser for cooling the sample of steam is that it is easier to separate air from a cooled steam sample.

In some embodiments, the air detection circuit of the autoclave according to the invention may be connected to the chamber via a restrictor to slow the rate of flow of the sample of steam. One advantage of such an arrangement is that the condenser of the air detection circuit has more time to cool the steam sufficiently such that the air detector can separate the air. A further advantage is that a slowed sample of steam takes more time to pass through the air detector, improving the separation of air from the steam.

In some embodiments, the autoclave may comprise a waste water tank to which the air detector is connected such that water, preferably degassed water, from the waste water tank can be used to fill the air detection circuit prior to use. One advantage of this feature is that fresh water is not used to prime the air detection circuit. Fresh water is generally required to meet sterilisation standards and can be expensive. A further advantage is that a volume of water may be used at least twice in the autoclave: firstly to generate steam and secondly to prime the air detection circuit.

In some embodiments, the air detector of the autoclave may be connected to the waste water tank via a valve. One advantage of this feature is that the air detector can be isolated from the waste water tank when the air detection circuit is emptied.

In some embodiments, the autoclave may comprise a feed water tank and the condenser may cool the steam by exchanging heat between the sample of steam and the feed water. In some embodiments, the cooling step of the method of the invention comprises exchanging heat with water to be used in the generation of steam. One advantage of exchanging heat between the sample of steam and water to be used in the generation of steam or feed water is that heat from the steam is recyclable so that the energy input to run the autoclave is reduced, thereby improving efficiency and reducing the cycle time for the autoclave because the feed water takes less time to heat to steam.

In some embodiments, the method of the invention may comprise a step of providing a condenser for cooling the sample of steam. In a further embodiment, the emptying step of the method of the invention may include emptying the condenser and the filling step may include filling the condenser with water, preferably degassed water.

In some embodiments, the providing step of the method of the invention may include providing an air detector having a detection column and the method may further comprise a displacing step of allowing the separated air to displace water in the detection column. The displacing step may be performed after the separating step. In such embodiments, the detecting step of the method of the invention may comprise detecting whether a level of water in the detection column has fallen below a pre-determined level.

In some embodiments, the method of the invention further comprises the step of sending out a signal that the pre-determined volume of air is present in the air detector. In some embodiments, the autoclave according to the invention comprises an alarm for sending out a signal that the pre-determined volume of air is present in the air detector. The signal may be an auditory or visual signal. One advantage of sending out a signal when a sterilisation cycle has failed is that it alerts a user of the autoclave.

According to the present invention there is further provided an air detector which comprises:
a separation vessel and
a detection column;
wherein the detection column comprises a plurality of lower level sensors and an upper level sensor for the presence of water. One advantage of such an air detector is that by having more than one lower level sensor, the plurality of sensors automatically provide back up for each other, should one fail. This is a safety feature because it avoids the generation of false results in the functioning of the autoclave, e.g. of a failed cycle being identified as a pass which, in the worst case, might mean that an unsterilized piece of equipment is used in a surgical intervention. In one embodiment, the plurality of sensors may be a first and second lower level sensor.

The invention will now be illustrated by way of example without limiting the scope of the claims by reference to the following Figures of the drawings:
**Figure 1** shows a schematic cross-sectional view of an air detector according to the invention; and
**Figure 2** shows a schematic view of an autoclave according to the invention comprising an air detection circuit according to the invention.

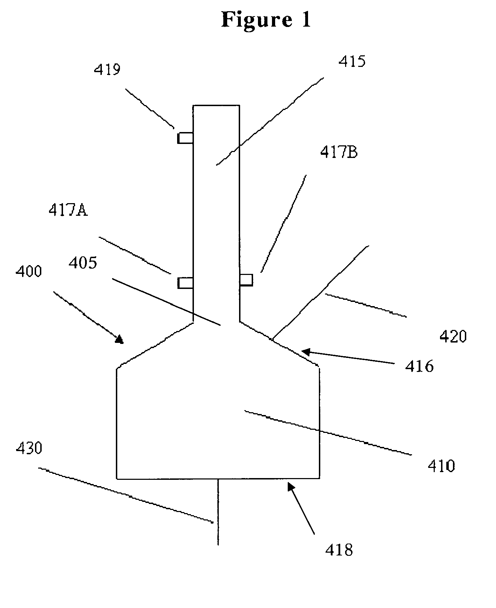

The air detector 400 shown in Figure 1 has a separation vessel 410 and a detection column 415. Separation vessel 410 has an upper part indicated generally at 416 and a base indicated generally at 418. Separation vessel 410 has an inlet 420 formed in its upper part 416, an outlet 430 formed in its base 418 and an opening 405 in its upper part 416 to which detection column 415 is connected.

Detection column 415 is arranged vertically above the separation vessel 410. The dimensions of opening 405 and detection column 415 are chosen to ensure that air separates from any steam in the separation vessel 410. Detection column 415 has a first lower level sensor 417A, a second lower level sensor 417B and an upper level sensor 419. Typically, each sensor 417A,417B,419 is suitable for detecting the presence of water. The upper level sensor 419 is located proximal to the top of the detection column 415. The first and second lower level sensors 417A,417B are proximal to the opening 405 of separation vessel. The position of the sensors 417A,417B within the detection column 415 influences the amount of air which may be detected by the air detector 400. In an alternative embodiment, the first and the second air detector lower level sensors 417A,417B may be located at middle length of the detection column 415.

Prior to use, the air detector 400 is primed by introducing a priming volume of water into separation vessel 410 through either inlet 420 or outlet 430. The priming volume of water has a volume which is sufficient to fill air detector 400. In an alternative embodiment, the priming volume of water may be sufficient to fill the air detection circuit 100.

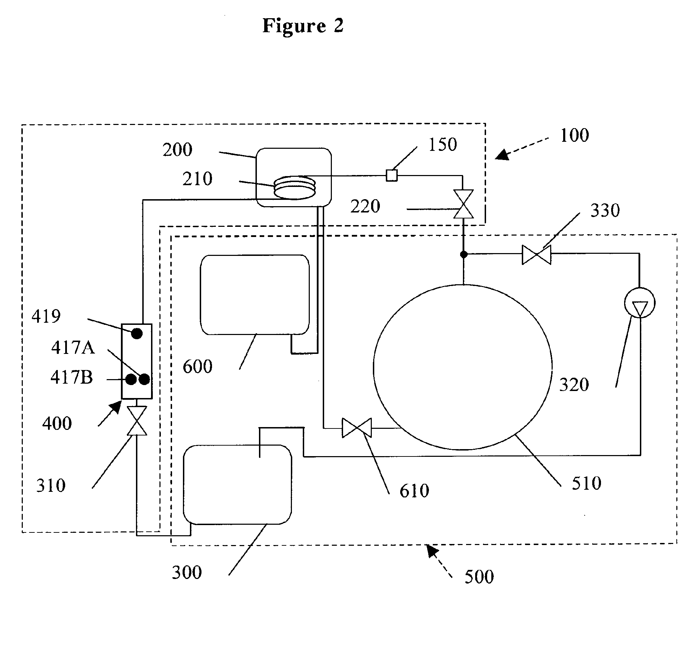

In use, in order to determine whether a detectable volume of air is present in a volume of steam, the air detector is operated as follows. A sample volume of steam is passed through the air detector 400 by introducing it into separation vessel 410 through inlet 420. Whilst the steam is in separation vessel 410, any air entrained with the steam separates and is trapped in detection column 415. The separated volume of air is a detectable volume of air if the level of water in detection column 415 falls to below that of first and second lower level sensors 417A,417B. Outlet 430 connects the air detector 400 to the air detection circuit 100 shown in Figure 2.

In Figure 2, there is schematic view of an autoclave 500 according to the invention comprising an air detection circuit 100 according to the invention. The autoclave indicated generally at 500 has a feed water tank 600, a heat exchange tank 200, a feed water valve 610, a chamber 510, a chamber vent valve 330, a vacuum pump 320 and a waste water tank 300. The feed water tank 600 is in fluid communication with the heat exchange tank 200 which is in fluid communication with the chamber 510 via feed water valve 610. Chamber 510 is in fluid communication with vacuum pump 320 via the chamber vent valve 330. Vacuum pump 320 is in fluid communication with waste water tank 300. Thus, the feed water tank 600, heat exchange tank 200, feed water valve 610, chamber 510, chamber vent valve 330, vacuum pump 320 and waste water tank 300 are connected in series. Where it is said that a feature is in fluid communication with another feature, it should be understood that the two features are connected by a fluid-tight pipe or conduit.

In use, feed water tank 600 contains clean feed water for a number of cycles of the autoclave 500. The heat exchange tank 200 is arranged so that it can be fed by gravity from feed water tank 600. In use, it contains clean feed water from feed water tank 600 to cool steam from chamber 510 during operation of the air detection circuit 100. Feed water valve 610 controls the flow of clean feed water from heat exchange tank 200 into the chamber 510.

In use, chamber 510 contains a load to be sterilised and has a door (not shown) to allow insertion and removal of the load. Chamber 510 also has a boiler (not shown) for the generation of steam for use in the operation of the autoclave 500. In an alternative embodiment, an external boiler may be used. Vacuum pump 320 removes air and/or water and/or steam from chamber 510 to reduce the pressure in the chamber 510 (e.g. to below atmospheric pressure). Vacuum pump 320 may assist in a drying cycle of the autoclave 500. Waste water tank 300 contains used water or degassed water from a number of cycles of the autoclave.

Air detection circuit 100 is in fluid communication with chamber 510 and waste water tank 300 of autoclave 500. Air detection circuit 100 has a vacuum valve 220, a restrictor 150, a condenser 210, an air detector 400 and an air detector fill valve 310. The vacuum valve 220 is in fluid communication with chamber 510 and restrictor 150. Restrictor 150 is in fluid communication with vacuum valve 220 and condenser 210. The condenser 210 is in fluid communication with restrictor 150 and air detector 400. Air detector 400 is in fluid communication with condenser 210 and air detector fill valve 310. Air detector fill valve 310 is in fluid communication with air detector 400 and waste water tank 300. Thus the vacuum valve 220, restrictor 150, condenser coil 210, air detector 400 and air detector fill valve 310 are connected in series.

Vacuum valve 220 controls the flow of air and/or water and/or steam from chamber 510 into the air detection circuit 100 or from air detection circuit 100 to vacuum pump 320 via chamber vent valve 330. The restrictor 150 slows the flow of steam from chamber 510 and vacuum valve 220 to such a rate so as the condenser 210 can cool the steam sufficiently for the air detector to separate any entrained air.

The condenser 210 is placed in heat exchange tank 200 such that when steam is passed through condenser 210, it is cooled by the clean feed water in heat exchange tank 200. Air detector 400 allows separation of air from a sample of cooled steam from a sterilisation cycle of autoclave 100. Air detector 400 includes one or more sensors for detecting whether the volume of separated air is a detectable volume of air. Air detector 400 may be an air detector as shown in Figure 1. In an alternative embodiment, a different air detector 400 may be used such as that shown in Figure 4 of EP-A-1 867 344 and described in paragraph 35 of that publication. Air detector fill valve 310 controls flow of a priming volume of degassed water into the air detector circuit 100 from waste water tank 300. A priming volume of degassed water may be a volume sufficient to fill the air detector 400 or a volume sufficient to fill the air detector circuit 100. Air detector fill valve 310 also allows the passage of cooled separated steam from the air detector 400 into waste water tank 300.

In use, the air detection circuit 100 is used with the autoclave 500 to detect the presence of air during a sterilisation cycle by performing a priming cycle before the sterilisation cycle of the autoclave 500 and a measurement cycle after the sterilisation cycle. The priming cycle comprises three steps which are emptying the air detector 400, filling the air detection circuit 100 with water and validating the priming of the air detector 400.

In the emptying step, the air detector 400 is emptied of any residual water and/or air by operating vacuum pump 320 with chamber vent valve 330 and vacuum valve 220 open and air detector fill valve 310 closed. In this first priming step, the contents of air detector 400 are drawn out of air detector 400 through restrictor 150, condenser 210, vacuum valve 220 and chamber vent valve 330 to the vacuum pump 320 which pumps the contents of the air detection circuit 100 into waste water tank 300. The first priming step is performed until a priming pressure has been reached. The first priming step may be performed at the same time as when a vacuum is drawn in chamber 510 as part of a pre-sterilisation cycle in the operation of autoclave 500 such that the priming steps for the air detection circuit 100 do not substantially lengthen the cycle time of the autoclave 500.

In the filling step of the priming cycle, air detector fill valve 310 is opened to draw a priming volume of degassed water from the waste water tank 300 into air detector 400. This is done whilst the vacuum pump 320 is operational such that any water drawn through air detection circuit 100 during the filling step is immediately drawn through chamber vent valve 330 by the action of vacuum pump 320 and not into chamber 510. During the filling step, air detector 400 is filled with degassed water. The advantage of using degassed water from the waste water tank to prime the air detector 400 is that air detection circuit 100 is more sensitive because any air detected during the measurement cycle is from the use of the autoclave 500, not from air entrained in the priming volume of water. After a priming period of time, vacuum valve 220 and air detector fill valve 310 are closed.

In the validation step of the priming cycle, sensors 417A,417B,419 are operated to detect the presence of water. If all sensors 417A,417B,419 do not detect the presence of water at the end of the priming steps, the priming cycle has failed. The cycle failure will then be communicated to a user of autoclave 500, e.g. by use of an auditory alarm or by a visual signal for example on a display panel (not shown) on the autoclave 500. For a successful priming of the air detection circuit 100, the air detector 400 and associated pipe work are filled with water.

After the priming cycle is complete, the autoclave 500 is operated to perform a sterilisation cycle in the usual way. A person of skill in the art would easily be able to determine the manner in which such a cycle should be performed. At the end of the sterilisation cycle and before or during the performing of the post-sterilisation cycle of cooling the chamber 510 and emptying it of steam, the following measurement cycle is performed to determine whether a failure volume of air was present in chamber 510 during the sterilisation cycle. A failure volume of air is sufficient air for the sterilisation cycle to have failed to ensure complete sterilisation of the contents of chamber 510. The air detector 400 is arranged such that the detectable volume of air (determined by the dimensions of detection column 415 and the placing of sensors 417A,417B) is less than the failure volume. The measurement cycle comprises five steps which are the steps of taking a sample optionally containing, cooling the sample, separating any air from the sample, disposing of the sample, measuring the separated air.

The sample taking step of the measurement cycle comprises taking a sample volume of steam and optionally air from the chamber 510 by opening vacuum valves 220 and 310 for a sampling period of time. In one embodiment, the size of the sample volume of steam and optionally air is selected such that the ratio of the detectable volume of air to the sample volume is proportional to the ratio of the failure volume of air to the volume of chamber 510. In an alternate embodiment, the time taken for the detection column 415 to be filled with air is used to determine whether a sterilisation cycle is successful or not. This is because the rate of filling of the detection column 415 may be a function of the amount of air in the chamber 510 after a sterilisation cycle. In such an embodiment, the sampling period of time is chosen to be sufficient to measure the rate of both successful and unsuccessful sterilisation cycles. Once the sample of steam and optionally air has been taken from chamber 510, vacuum valves 220 and 310 are closed and the post-sterilisation cycle is performed according to any procedure known a to person of skill in the art.

The cooling step of the measurement cycle comprises passing the sample of steam and optionally air through condenser 210 where it is cooled by the water in heat exchange tank 200. Thus the steam pre-heats water in heat exchange tank 200 before it is used in a pre-sterilisation or sterilisation cycle thereby improving the efficiency of autoclave 500 by recycling energy. In operation of autoclave 500, a volume of water is generally used in three ways: to cool a sample volume of steam, to form steam for use in a sterilisation cycle and to prime the air detector 400. As no steam comes into contact with heat exchange tank 200, there is greater flexibility about what material can be used in its construction because it does not have to be able to withstand such a great variation in temperature.

The air separation step of the measurement cycle comprises passing the cooled sample of steam and optionally air into the air detector 400 where any air entrained in the cooled steam separates and rises up into detection column 415. The separated air in detection column 415 displaces the water in the air detection column 415 downwards.

The disposal step of the measurement cycle comprises passing the cooled steam through open air detector fill valve 310 into waste water tank 300.

The measurement step of the measurement cycle comprises operating sensors 417A,417B to detect the presence of water. If sensors 417A,417B do not detect the presence of water, the separated air in detection column 415 will have displaced the water in air detection column 415 downwards below the level of sensors 417A,417B such that the sensors 417A,417B are uncovered. A cycle failure will then be communicated to a user of autoclave 500, e.g. by use of an audible alarm or by a visual signal for example on a display panel (not shown) on the autoclave 500.

The autoclave 500 includes a programmable air detector circuit controller (not shown) for controlling the operation of the air detector circuit 100. The operation of the air detector circuit 100 is determined by the following variables: the priming pressure which is used to determine the end of the emptying step of the priming cycle; the priming period of time which is used to determine the length of time during which the vacuum valve 220 and air detector fill valve 310 are open during the filling step of the priming cycle; the sample volume of steam and optionally air used in the measurement cycle. Depending on the type of load used during the sterilisation cycle, the value of one or more of the variables may be changed. One advantage of the invention is that the air detection circuit is under the control of a programmable air detector circuit controller such that the variables can be changed, where appropriate.

## Claims

1. An autoclave having an air detection circuit for use in detecting whether a pre-determined volume of air is present in a sample of steam wherein the autoclave comprises a chamber and a vacuum pump and wherein the air detection circuit is operatively connectable to the chamber and comprises:
an air detector for receiving the sample of steam, the air detector having a separator for separating air from the sample of steam and at least one sensor for detecting whether the air separated from the sample of steam has the predetermined volume;
wherein the autoclave is arranged such that the air detection circuit is operatively connectable to the vacuum pump such that the vacuum pump can be used to empty the contents, if any, in the air detection circuit.

2. An autoclave according to Claim 1 wherein the air detection circuit comprises a condenser for cooling the sample of steam.

3. An autoclave according to Claim 1 or Claim 2 wherein the air detection circuit includes a restrictor to slow the rate of flow of the sample of steam.

4. An autoclave according to any one of the preceding claims wherein the autoclave comprises a waste water tank to which the air detector is operatively connectable such that water or degassed water from the waste water tank can be used to fill the air detection circuit prior to use.

5. An autoclave according to Claim 4 wherein the air detector is operatively connectable to the waste water tank via a valve.

6. An autoclave according to any one of the preceding claims wherein the autoclave comprises a tank containing feed water for the autoclave and wherein the condenser cools the steam by exchanging heat between the sample of steam and the feed water.

7. A method of detecting the presence of a pre-determined volume of air in a sample of steam, comprising the steps of:
providing an air detector adaptable to detect whether a predetermined volume of air is present;
emptying the contents, if any, in the air detector;
passing a sample of steam through the air detector;
separating any air from the sample of steam in the air detector; and
detecting whether the separated air has the pre-determined volume.

8. A method according to Claim 7 further comprising the step of filling the air detector with water or degassed water before the passing step.

9. A method according to Claim 7 or Claim 8 further comprising the step of cooling the sample of steam.

10. A method according to Claim 9 further comprising the step of:
providing a condenser for cooling the sample of steam, preferably wherein the emptying step includes emptying the condenser;
and more preferably further comprising the step of:
filling the condenser with water or degassed water.

11. A method according to any one of claims 7 to 10 wherein the providing step includes providing the air detector with a detection column and wherein the method comprises a displacing step of allowing the separated air to displace water in the detection column.

12. A method according to Claim 11 wherein the detecting step comprises detecting whether a level of water in the detection column has fallen below a pre-determined level.

13. A method according to any one of claims 8 to 12 wherein the cooling step comprises exchanging heat with water to be used in the generation of steam.

14. A method according to any one of claims 8 to 13 further comprising the step of:
sending out a signal that the pre-determined volume of air is present in the air detector.

15. An air detector which comprises:
a separation vessel; and
a detection column extending from the separation vessel;
wherein the detection column comprises a plurality of lower level sensors and an upper level sensor to detect the presence of water; preferably the plurality of sensors are a first and second lower level sensor.
